# FASCICULE DE BREVET EUROPEEN

(11) **EP 3 563 916 B1**
(45) Date de publication et mention de la délivrance du brevet: **17.03.2021**
(21) Numéro de dépôt: 19169111.2
(22) Date de dépôt: 12.04.2019
(51) Int. Cl.: B01D 3/10, B01J 19/24, C07C 7/04, C10G 45/58, C07C 15/04, C07C 15/06, B01D 3/00, B01D 3/14, C07C 7/00, C10G 7/00, C10G 29/20

(54) **PROCEDE ET DISPOSITIF DE SEPARATION D'AROMATIQUES INVERSE**
VERFAHREN UND VORRICHTUNG ZUR UMKEHRTRENNUNG VON AROMASTOFFEN
METHOD AND DEVICE FOR INVERSE SEPARATION OF AROMATICS

(30) Priorité: 04.05.2018 FR 1853885
(43) Date de publication de la demande: 06.11.2019
(73) Titulaire: AXENS, 92508 Rueil-Malmaison Cedex (FR)
(72) Inventeur: PIGOURIER, Jérôme, 92190 MEUDON (FR); PREVOST, Isabelle, 92500 RUEIL MALMAISON (FR)
(74) Mandataire: IFP Energies nouvelles

(56) Documents cités:
- FR-A1- 2 998 301
- US-A1- 2014 257 010

## Description

### Domaine technique

La présente invention s'inscrit dans le domaine des procédés et dispositifs de séparation de composés aromatiques. L'objet selon l'invention s'applique en particulier au cas d'un complexe aromatique faisant appel à une séparation par distillation entre du benzène, du toluène et des composés plus lourds à 8 atomes de carbones ou plus (notés ci-après, composés C8+).

### Etat de l'art

Le brevet FR2998301 B1 décrit une méthode permettant un gain énergétique global sur la consommation de combustible et d'électricité d'un complexe aromatique pour la séparation par distillation entre du benzène, du toluène et des composés C8+. Spécifiquement, le principe de ladite méthode réside dans la génération de vapeur basse pression au niveau de certaines colonnes, la vapeur basse pression ainsi générée étant utilisée comme fluide caloporteur, avec ou sans compression intermédiaire, par exemple pour le rebouillage à plus basse température d'autres colonnes ayant subi un abaissement de pression.

### Résumé

Dans le contexte précédemment décrit, un premier objet de la présente invention est de permettre la réduction de la quantité d'énergie requise pour réaliser la séparation entre le benzène, le toluène et des composés C8+.

Selon un premier aspect, l'objet précité, ainsi que d'autres avantages, sont obtenus par un procédé de séparation d'une charge comprenant du benzène, du toluène et des composés à 8 atomes de carbone ou plus, dans un dispositif de séparation comprenant au moins une colonne de reformat, une unité d'extraction des aromatiques et une unité de transalkylation, les effluents desdites unités étant séparés dans les colonnes à distiller suivantes : colonne de benzène, colonne de toluène et colonne de stabilisation, ledit procédé comprenant les étapes suivantes :
on alimente directement la colonne de toluène avec une coupe C7+ issue du fond de la colonne de stabilisation disposée en aval de l'unité de transalkylation pour soutirer un produit de tête de la colonne de toluène enrichi en benzène et en toluène et un produit de fond de la colonne de toluène enrichi en composés à 8 atomes de carbone ou plus,
on alimente la colonne de benzène avec le produit de tête de la colonne de toluène pour soutirer un produit de tête de la colonne de benzène enrichi en benzène et un produit de fond de la colonne de benzène enrichi en toluène, et
on alimente l'unité de transalkylation avec le produit de fond de la colonne de benzène enrichi en toluène,
dans lequel on alimente la colonne de toluène avec une coupe essentiellement aromatique issue de l'unité d'extraction des aromatiques, l'alimentation de la colonne de toluène issue de l'unité d'extraction des aromatiques étant effectuée séparément et au-dessus de l'alimentation de la colonne de toluène issue du fond de la colonne de stabilisation, ou
dans lequel on alimente la colonne de benzène avec la coupe essentiellement aromatique issue de l'unité d'extraction des aromatiques en mélange avec ou séparément du produit de tête de la colonne de toluène.

Selon un ou plusieurs modes de réalisation, on alimente en outre la colonne de toluène avec un produit de tête de la colonne de purification en mélange avec ou séparément de la coupe C7+ issue du fond d'une colonne de stabilisation.

Selon un ou plusieurs modes de réalisation, on alimente en outre l'unité de transalkylation avec un produit de tête d'une colonne de purification en mélange avec ou séparément du produit de fond de la colonne de benzène.

Selon un ou plusieurs modes de réalisation, le dispositif de séparation comprend en outre une unité de séparation du paraxylène et une unité d'isomérisation des xylènes, lesdits effluents étant séparés dans les colonnes à distiller additionnelles suivantes : colonne des xylènes, colonne des aromatiques lourds, colonne de raffinat, colonne d'extrait, colonne de purification, déheptaniseur et stripeur.

Selon un ou plusieurs modes de réalisation, seulement la colonne des xylènes et la colonne des aromatiques lourds alimentent l'unité de transalkylation en composés aromatiques en C9+.

Selon un ou plusieurs modes de réalisation, on alimente la colonne de reformat avec la charge pour produire une coupe C7- en tête de la colonne de reformat, et on alimente l'unité d'extraction des aromatiques avec la coupe C7- pour extraire des composés paraffiniques de la coupe C7- et produire la coupe essentiellement aromatique.

Selon un ou plusieurs modes de réalisation, les conditions opératoires de la colonne de benzène C9 sont les suivantes :
température de 130°C à 180°C au rebouilleur, de 70°C à 130°C au condenseur ;
pression : entre 0,1 Mpa et 0,5 Mpa ; et
entre 35 et 55 plateaux théoriques.

Selon un ou plusieurs modes de réalisation, les conditions opératoires de la colonne de toluène C10 sont les suivantes :
température de 130°C à 260°C au rebouilleur, de 50°C à 200°C au condenseur ;
pression : entre 0,05 Mpa et 1 Mpa ; et
entre 30 et 55 plateaux théoriques.

Selon un deuxième aspect, l'objet précité, ainsi que d'autres avantages, sont obtenus par un dispositif de séparation d'une charge comprenant du benzène, du toluène et des composés à 8 atomes de carbone ou plus, le dispositif de séparation comprenant au moins une colonne de reformat, une unité d'extraction des aromatiques et une unité de transalkylation, le dispositif de séparation comprenant en outre les colonnes suivantes pour la distillation des effluents desdites unités : colonne de benzène, colonne de toluène et colonne de stabilisation,
la colonne de toluène étant adaptée pour être alimentée avec une coupe C7+ issue du fond de la colonne de stabilisation disposée en aval de l'unité de transalkylation et pour distribuer un produit de tête de la colonne de toluène enrichi en benzène et en toluène et un produit de fond de la colonne de toluène enrichi en composés à 8 atomes de carbone ou plus,
- la colonne de benzène étant adaptée pour être alimentée avec le produit de tête de la colonne de toluène et pour distribuer un produit de tête de la colonne de benzène enrichi en benzène et un produit de fond de la colonne de benzène enrichi en toluène,
- l'unité de transalkylation étant adaptée pour traiter le produit de fond de la colonne de benzène enrichi en toluène,
dans lequel la colonne de toluène est adaptée pour être alimentée avec une coupe essentiellement aromatique issue de l'unité d'extraction des aromatiques, l'alimentation de la colonne de toluène issue de l'unité d'extraction des aromatiques étant effectuée séparément et au-dessus de l'alimentation de la colonne de toluène issue du fond de la colonne de stabilisation, ou
dans lequel la colonne de benzène est adaptée pour être alimentée avec la coupe essentiellement aromatique issue de l'unité d'extraction des aromatiques en mélange avec ou séparément du produit de tête de la colonne de toluène.

Selon un ou plusieurs modes de réalisation, la colonne de toluène est adaptée pour être alimentée avec un produit de tête d'une colonne de purification en mélange avec ou séparément de la coupe C7+ issue du fond de la colonne de stabilisation.

Selon un ou plusieurs modes de réalisation, l'unité de transalkylation est adaptée pour être alimentée avec un produit de tête d'une colonne de purification en mélange avec ou séparément du produit de fond de la colonne de benzène.

Selon un ou plusieurs modes de réalisation, le dispositif de séparation comprend en outre une unité de séparation du paraxylène, une unité d'isomérisation des xylènes et les colonnes additionnelles suivantes pour la distillation desdits effluents : colonne des xylènes, colonne des aromatiques lourds, colonne de raffinat, colonne d'extrait, colonne de purification, déheptaniseur et stripeur.

Selon un ou plusieurs modes de réalisation, le dispositif de séparation est adapté pour alimenter la colonne de reformat avec la charge pour produire une coupe C7- en tête de la colonne de reformat, et alimenter l'unité d'extraction des aromatiques avec la coupe C7-pour extraire des composés paraffiniques de la coupe C7- et produire la coupe essentiellement aromatique.

Selon un ou plusieurs modes de réalisation, les conditions opératoires de la colonne de benzène C9 sont les suivantes :
température de 130°C à 180°C au rebouilleur, de 70°C à 130°C au condenseur ;
pression : entre 0,1 Mpa et 0,5 Mpa ; et
entre 35 et 55 plateaux théoriques.

Selon un ou plusieurs modes de réalisation, les conditions opératoires de la colonne de toluène C10 sont les suivantes :
température de 130°C à 260°C au rebouilleur, de 50°C à 200°C au condenseur ;
pression : entre 0,05 Mpa et 1 Mpa ; et
entre 30 et 55 plateaux théoriques.

Des modes de réalisation du procédé et du dispositif référencés ci-dessus ainsi que d'autres caractéristiques et avantages vont apparaître à la lecture de la description qui va suivre, donnée à titre uniquement illustratif et non limitatif, et en référence aux dessins suivants.

### Brève description des dessins

La figure 1 décrit un schéma d'un complexe aromatique comprenant une section de séparation F de référence pour la séparation de benzène, de toluène et des composés C8+.
La figure 2 décrit un schéma d'un complexe aromatique comprenant une section de séparation F selon l'invention pour la séparation de benzène, de toluène et de composés C8+.

### Description détaillée

L'invention concerne le domaine des procédés et dispositifs pour la séparation d'une charge comprenant du benzène, du toluène et des composés C8+ (e.g. C8 à C10) pouvant notamment comprendre du paraxylène.

Le procédé et dispositif de séparation selon l'invention peut être défini comme une série d'étapes et sections de conversion et de séparation destinée à séparer benzène/toluène/xylènes et/ou convertir du toluène en composés C8+ et notamment des composés aromatiques à huit atomes de carbone, appelé xylènes, et plus particulièrement le paraxylène, à partir d'une charge riche en composés aromatiques allant du benzène à des composés aromatiques à plus de 10 atomes de carbone (notés C10+), provenant par exemple d'une unité de reformage catalytique. La charge riche en composés aromatiques présente typiquement des teneurs en composés soufrés, azotés et oléfines très faible à nulle (e.g. teneur en soufre < 0,5 ppm poids et/ou teneur en azote < 0,5 ppm poids et/ou indice de brome (bromine number en anglais) < 1g/100g selon ASTM D1159), car ces composés peuvent affecter les performances et la durée de vie de certains catalyseurs et des tamis moléculaires mis en œuvre dans les unités du complexe aromatique.

Le premier objet selon la présente invention peut être défini comme une inversion de l'ordre des colonnes de benzène et de toluène combinée à des alimentations distinctes des charges des colonnes (qui étaient dirigées en mélange vers la colonne de benzène selon le brevet FR2998301 B1), ceci pour une meilleure efficacité énergétique.

Dans la description qui suit on parle de « *complexe »* pour désigner tout dispositif de raffinage ou de pétrochimie comportant au moins deux colonnes à distiller. Cette définition est très large et comprend, par exemple, le dispositif de craquage catalytique des essences et le dispositif de production de paraxylène ou de métaxylènes à partir de coupes aromatiques dit « *complexe aromatique ».* La description qui suit et l'exemple qui illustre le procédé et le dispositif de séparation selon l'invention sont donnés dans le cas d'un complexe aromatique, mais il est bien entendu qu'un complexe aromatique ne constitue qu'un cas d'application et ne limite en aucun cas la portée du procédé et du dispositif de séparation exposés dans la présente description.

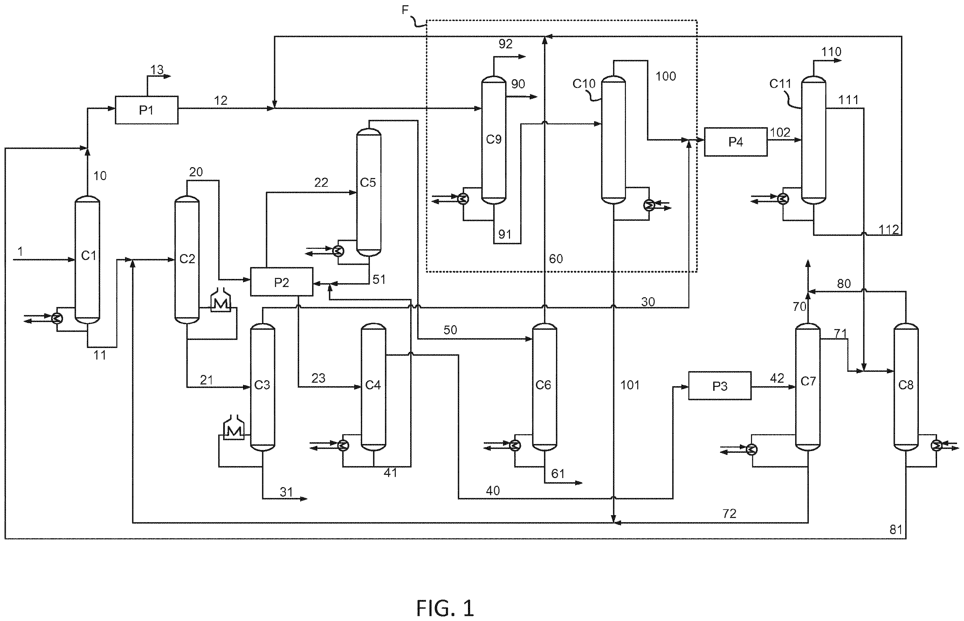

La figure 1 décrit un schéma d'un procédé et d'un complexe aromatique de référence pour la séparation de benzène, de toluène et de composés C8+.

En référence à la figure 1, il est notamment décrit un enchaînement de deux colonnes, la colonne de benzène C9 et la colonne de toluène C10. Cet enchaînement de colonnes est alimenté par le mélange d'une coupe C6-C7 essentiellement aromatique issue de l'unité d'extraction des aromatiques P1 et d'une coupe C7+ issue du fond de la colonne de la colonne de stabilisation C11 en aval de l'unité de transalkylation P4. La colonne de benzène C9 produit en tête du benzène en tant que produit final et en fond une coupe C7+ envoyée vers la colonne de toluène C10. Le toluène est récupéré en tête de colonne de toluène C10, tandis qu'une coupe C8+ est extraite en fond de la colonne de toluène C10.

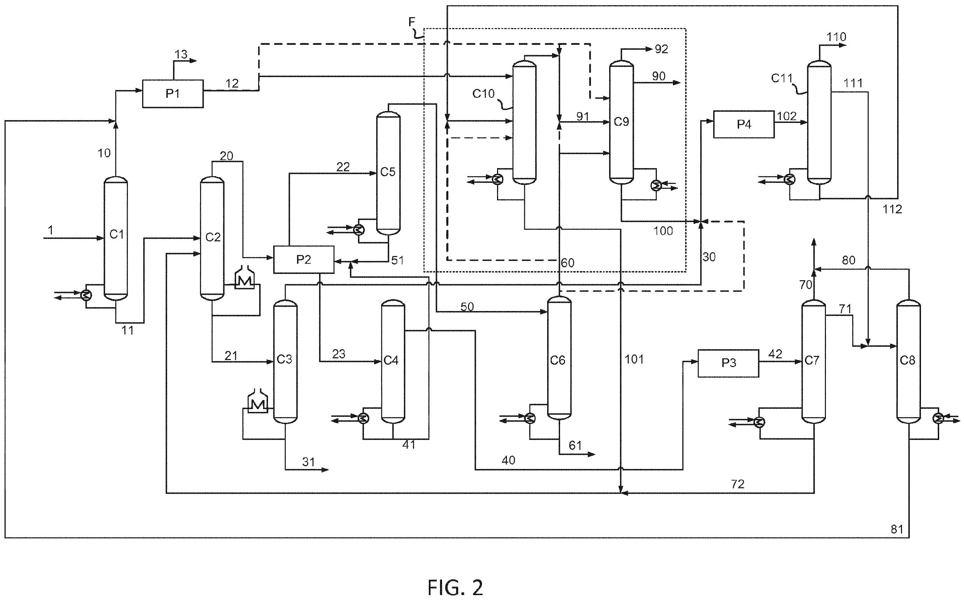

La figure 2 décrit un schéma d'un procédé et dispositif de séparation selon un ou plusieurs modes de réalisation de la présente invention, permettant notamment de diminuer la consommation énergétique nécessaire à la séparation entre le benzène, le toluène et les C8+ par rapport au procédé et complexe aromatique de référence.

En référence à la figure 2, il est notamment décrit des zones du procédé et du dispositif de séparation qui ont été modifiées de sorte que la colonne de toluène (C10) est alimentée avec une coupe C7+ issue du fond de la colonne de stabilisation (C11) disposée en aval de l'unité de transalkylation (P4) pour soutirer un produit de tête de la colonne de toluène (C10) enrichi en benzène et en toluène et un produit de fond de la colonne de toluène (C10) enrichi en composés à 8 atomes de carbone ou plus ; la colonne de benzène (C9) est alimentée avec le produit de tête de la colonne de toluène (C10) pour soutirer un produit de tête de la colonne de benzène (C9) enrichi en benzène et un produit de fond de la colonne de benzène (C9) enrichi en toluène ; et l'unité de transalkylation (P4) est alimentée avec le produit de fond de la colonne de benzène (C9) enrichi en toluène. Selon un ou plusieurs modes de réalisation, la colonne de toluène (C10) est également alimentée avec une coupe essentiellement aromatique (notée ci-après coupe C6-C7) issue de l'unité d'extraction des aromatiques (P1), l'alimentation de la colonne de toluène (C10) issue de l'unité d'extraction des aromatiques (P1) étant effectuée séparément et au-dessus (en aval) de l'alimentation de la colonne de toluène (C10) issue du fond de la colonne de stabilisation (C11). Selon un ou plusieurs modes de réalisation, la colonne de benzène (C9) est également alimentée avec la coupe C6-C7 issue de l'unité d'extraction des aromatiques (P1) en mélange avec ou séparément du produit de tête de la colonne de toluène (C10).

Les étapes et sections de conversion et de séparation du procédé et dispositif de séparation selon l'invention sont décrites plus en détails ci- après.

Lorsque l'objectif est uniquement de séparer le benzène et les composés en C8 et éventuellement le toluène d'une charge, seules les étapes ou sections C1, P1, C9, C10, P4 et C11 sont présentes. Les autres sections décrites ci-après ne sont utiles que lorsque l'on souhaite séparer les xylènes, voire maximiser le rendement en paraxylène.

### Colonne de reformat C1

La charge à traiter est envoyée via la ligne 1 vers la première colonne à distiller notée colonne de reformat C1 qui sépare le toluène et les composés plus légers (coupe de composés C7-) des composés plus lourds (coupe de composés C8 à C10+).

### Unité d'extraction des aromatiques P1

L'effluent de tête de la colonne de reformat C1 est dirigé vers l'unité d'extraction des aromatiques P1 via la ligne 10.

Le toluène et optionnellement les composés récupérés en fond du stripeur C8 sont envoyés via la ligne 81 à l'unité d'extraction des aromatiques P1.

L'unité d'extraction des aromatiques P1 sépare la coupe C6-C7, essentiellement aromatique, d'un produit comprenant des composés paraffiniques envoyé à l'extérieur du complexe aromatique via la ligne 13. Le solvant utilisé préférentiellement dans l'unité d'extraction des aromatiques P1 est la N-formylmorpholine (NFM).

En référence à la figure 1, la coupe C6-C7 issue de l'unité d'extraction des aromatiques P1 est dirigée au sein de la section de séparation F via la ligne 12 vers la colonne de benzène C9.

En référence à la figure 2, la coupe C6-C7 issue de l'unité d'extraction des aromatiques P1 est dirigée au sein de la section de séparation F via la ligne 12 soit vers la colonne de benzène C9, soit afin d'être purifiée de quelques C8 qu'elle pourrait contenir vers la colonne de toluène C10.

Selon un ou plusieurs modes de réalisation, l'unité d'extraction des aromatiques P1 comprend une unité de distillation extractive.

### Colonne des xylènes C2

Les composés aromatiques C8-C10+ récupérés au fond de la colonne C1 sont envoyés via la ligne 11 vers la colonne des xylènes C2 pour séparer les composés aromatiques en C9 et plus lourds (composés C9+) d'une coupe xylènes comprenant des composés aromatiques en C8 qui alimentent les unités du complexe aromatique situées en aval.

### Unité de séparation du paraxylène P2

La coupe xylènes, c'est à dire la coupe de composés aromatiques en C8 contenant du paraxylène, du métaxylène de l'orthoxylène et de l'éthylbenzène, est récupérée en tête de la colonne des xylènes C2 et est envoyée via la ligne 20 vers l'unité de séparation du paraxylène P2 qui récupère sélectivement le paraxylène contenu dans ladite coupe xylènes.

Ladite unité de séparation du paraxylène P2 peut être une unité d'adsorption du paraxylène, adaptée par exemple pour produire un mélange de paraxylène et de désorbant (appelé l'extrait) et un mélange des autres composés C8- aromatiques et de désorbant (appelé le raffinat).

L'adsorbant utilisé est un tamis moléculaire dédié à l'adsorption du paraxylène, c'est à dire qu'il présente une affinité particulièrement élevée envers ce composé.

Un solide adsorbant couramment utilisé est une zéolithe de type faujasite mise en forme avec un liant silicique échangée au baryum ou au potassium. Le désorbant préférentiellement utilisé est le paradiéthylbenzène (PDEB).

Selon un ou plusieurs modes de réalisation, l'unité de séparation du paraxylène P2 comprend une unité de cristallisation du paraxylène, par exemple telle que décrite dans le brevet US 3,467,724.

Selon un ou plusieurs modes de réalisation, l'unité de séparation du paraxylène P2 comprend une combinaison d'une unité d'adsorption du paraxylène et d'une unité de cristallisation telle que décrite par exemple dans le brevet EP-B- 053191.

### Colonne d'extrait C5

Cette colonne est utilisée lorsque l'unité de séparation est du type adsorption du paraxylène. Le flux d'extrait issu de l'unité d'adsorption du paraxylène et contenant le paraxylène et du désorbant est envoyé via la ligne 22 vers la colonne d'extrait C5 qui sépare le paraxylène du désorbant. Le désorbant récupéré au fond de la colonne d'extrait C5 est renvoyé vers la colonne d'adsorption via la ligne 51. Le paraxylène récupéré en tête de la colonne d'extrait C5 est envoyé vers la colonne de purification C6.

### Colonne de purification C6

Le flux de tête de la colonne d'extrait C5 est envoyé via la ligne 50 vers la colonne de purification C6 qui sépare le toluène (qui a été partiellement extrait avec le paraxylène) du paraxylène.

Le paraxylène de haute pureté produit est récupéré en fond de la colonne de purification C6 et conduit en tant que produit fini par pompage pour stockage via la ligne 61.

Selon un ou plusieurs modes de réalisation, le produit de tête de la colonne de purification C6 est dirigé vers la colonne de benzène C9 (ligne 60) au sein de la section de séparation F.

En référence à la figure 2, le produit de tête de la colonne de purification C6 peut être : dirigé soit vers la colonne de benzène C9, en mélange avec le flux de tête de la colonne C10 ou via une alimentation séparée, soit vers la colonne de toluène C10, par exemple en mélange avec la coupe C7+ issue du fond de la colonne de stabilisation C11 (ligne 112), afin de purifier ledit produit de tête de quelques C8 qu'il pourrait contenir ; et/ou recyclé directement vers l'unité de transalkylation P4, par exemple en mélange avec la coupe C7+ extraite en fond de la colonne de benzène C9.

### Colonne de raffinat C4

Le raffinat en provenance de l'unité de séparation du paraxylène P2 est envoyé via la ligne 23 vers la colonne de raffinat C4 qui sépare les C8 aromatiques (raffinat) du désorbant. Le désorbant récupéré en fond de la colonne C4 est renvoyé vers la section d'adsorption du paraxylène P2 via la ligne 41.

Le raffinat (coupe C8 aromatiques) est extrait par un soutirage latéral et envoyé via la ligne 40 comme charge de l'unité d'isomérisation des xylènes P3.

### Colonne de désorbant (non représentée)

Cette colonne est utilisée lorsque l'unité de séparation du paraxylène est du type adsorption du paraxylène. Une petite portion du désorbant circulant dans l'unité d'adsorption du paraxylène est envoyée vers la colonne de désorbant (non représentée) de manière à en éliminer les composés lourds qui sinon s'accumuleraient dans la boucle.

### Unité d'isomérisation des xylènes P3

L'unité d'isomérisation des xylènes P3 est utilisée pour convertir une charge appauvrie en paraxylène en un flux de xylènes à l'équilibre thermodynamique (noté « isomérat »).

Tout type de catalyseur susceptible d'isomériser les hydrocarbures à 8 atomes de carbones peut être utilisé dans le procédé et le dispositif de séparation selon la présente invention. De préférence, on utilise un catalyseur contenant un métal déshydrogénant comme le platine, le palladium ou le nickel et une phase acide, par exemple une alumine dopée, une zéolithe telle que la mordénite, la MFI, la zéolithe Y, ou les tamis moléculaires zéolitiques ou non zéolithiques comportant une acidité tels que les alumino phosphates (e.g. aluminophosphates AIPO, silicoaluminophosphates SAPO). On peut ainsi de manière plus préférée utiliser un catalyseur d'isomérisation comprenant une zéolithe de type structural EUO, telle que la zéolithe EUI, la zéolithe ZSM 50 ou la zéolithe TPZ3 telles que décrites dans les brevets US 4,640,829, EP-B-042226 ou EP- B-051318.

### Déheptaniseur C7

L'effluent de l'unité d'isomérisation des xylènes P3 est envoyé via la ligne 42 au déheptaniseur C7 qui sépare l'isomère (composés C8+ aromatiques) d'une coupe légère en C7- récupérée en tête de ladite colonne déheptaniseur C7. Cette coupe C7- est envoyée via la ligne 71 vers le stripeur C8 pour séparer les composés légers de la coupe C7.

La coupe C8+, formée des xylènes et des composés plus lourds, récupérée en fond du déheptaniseur C7 est recyclée via la ligne 72 vers la colonne des xylènes C2.

Compte tenu de la teneur en légers (C4-) importante dans le déheptaniseur C7, le produit de tête du déheptaniseur C7, peut comprendre une phase vapeur 70 (constituée majoritairement de composés légers (C4-) et une phase liquide 71, toutes deux issues du ballon de reflux (non représenté).

### Stripeur C8

Le stripeur (ou colonne de stripage) C8 est alimenté par la tête du déheptaniseur C7.

La coupe C7- stabilisée est récupérée en fond du stripeur C8 pour être envoyée à l'unité d'extraction des aromatiques P1 via la ligne 81.

Les composés légers (C4-) issus de la tête de stripper sont mélangés via la ligne 80 avec les légers issus de la tête du déheptaniseur (ligne 70) et purgés.

### Colonne des aromatiques lourds C3

Les composés aromatiques en C9+ récupérés en fond de la colonne des xylènes C2 sont envoyés via la ligne 21 vers la colonne des aromatiques lourds C3 qui sépare les composés aromatiques en C9 et C10 des composés plus lourds (tels que le naphtalène) qui peuvent avoir un effet défavorable sur le catalyseur de transalkylation et qui sont récupérés en fond via la ligne 31.

### Unité de transalkylation P4

Les composés aromatiques en C9 et C10 récupérés en tête de la colonne des aromatiques lourds C3 sont envoyés via la ligne 30 vers l'unité de transalkylation P4.

En référence à la figure 1, les composés aromatiques en C9 et C10 sont mélangés avec le toluène en provenance de la tête de la colonne de toluène C10.

En référence à la figure 2, les composés aromatiques en C9 et C10 sont mélangés avec le toluène en provenance du fond de la colonne de benzène C9.

L'unité de transalkylation P4 convertit le toluène et les composés aromatiques en C9+ provenant de la colonne de reformat C1 et de l'isomérat de l'unité d'isomérisation des xylènes P3 (après passage par la colonne des xylènes C2 et la colonne des aromatiques lourds C3) en un mélange de xylènes et de benzène via une réaction limitée par la thermodynamique. Selon un ou plusieurs modes de réalisation, la colonne des xylènes C2 et la colonne des aromatiques lourds C3 fournissent la majorité (substantiellement la totalité) des composés aromatiques en C9+ envoyés vers l'unité de transalkylation P4.

Tout type de catalyseurs de transalkylation est utilisable dans le procédé et le dispositif de séparation selon la présente invention, par exemple des catalyseurs à base de mordénite ou faujasite décrits dans le brevet US 3,437,710 ou les catalyseurs à base de zéolithes MCM-22 ou béta décrits dans le brevet US 5,030,787, ou les catalyseurs à base de zéolithes mordénite et MFI tels que décrits dans la demande de brevet US2012/0065446. Ces catalyseurs comprennent généralement en sus un composé métallique choisi de préférence parmi le groupe formé par le rhénium, le nickel, le cobalt, le molybdène, le tungstène, le palladium, et le platine.

### Colonne de stabilisation C11

L'effluent de l'unité de transalkylation P4 qui contient du benzène, le toluène non converti, et des aromatiques en C8 (e.g. xylènes) et en C9+ est envoyé via la ligne 102 vers la colonne de stabilisation C11 qui sépare les composés plus légers que le benzène, du benzène et des composés aromatiques plus lourds notés C7+.

Le gaz quittant (le ballon de reflux de) la colonne de stabilisation C11 est envoyé via la ligne 110 en limite du complexe aromatique.

Une coupe de benzène non purifié est soutirée latéralement et envoyée via la ligne 111 vers la colonne de stripage C8 qui permet de séparer les composés légers de ladite coupe. Selon un ou plusieurs modes de réalisation, la condensation partielle des gaz de tête de la colonne de stabilisation C11 est obtenue au moyen d'un aéroréfrigérant éventuellement suivi d'un réfrigérant à eau.

### Section de séparation F

La section de séparation F comprend la colonne de benzène C9 et la colonne de toluène C10.

### Colonne de benzène C9 selon le procédé et le complexe aromatique de référence

En référence à la figure 1, la coupe C6-C7 issue de l'unité d'extraction des aromatiques P1 (ligne 12) est mélangée avec la coupe C7+ issue du fond de la colonne de stabilisation C11 (ligne 112) et envoyée vers la colonne de benzène C9. A partir de la colonne de benzène C9, le benzène est extrait en tête en tant que produit final via la ligne 90. La coupe C7+ extraite en fond de la colonne de benzène C9 est dirigée via la ligne 91 vers la colonne de toluène C10.

### Colonne de toluène C10 selon le procédé et le complexe aromatique de référence

En référence à la figure 1, la colonne de toluène C10 est alimentée par la coupe C7+ issue du fond de la colonne de benzène C9. Le toluène récupéré en tête de la colonne de toluène C10 est dirigé via la ligne 100 comme charge de l'unité de transalkylation P4. La coupe C8+ extraite en fond de la colonne C10 est recyclée via la ligne 101 vers la colonne des xylènes C2.

### Colonne de toluène C10 selon le procédé et le dispositif de séparation selon la présente invention

En référence à la figure 2, la colonne de toluène C10 est alimentée par la coupe C7+ issue du fond de la colonne de stabilisation C11 (ligne 112). Le produit de tête de la colonne de toluène C10 est une coupe riche en composés C7- (e.g. essentiellement toluène + composés C6-) et le produit de fond une coupe C8+ riche en composés aromatiques à 8 atomes de carbones.

La coupe C8+ extraite en fond de la colonne de toluène C10 (*i.e.,* produit de fond de la colonne de toluène 10 enrichi en composés à 8 atomes de carbone ou plus) est recyclée via la ligne 101 vers la colonne des xylènes C2 qui sépare les C9+ et plus lourds de la coupe C8 aromatiques alimentant le dispositif de séparation du paraxylène.

Selon un ou plusieurs modes de réalisation, la coupe C6-C7 issue de l'unité d'extraction des aromatiques P1 est envoyée vers la colonne de toluène C10 de façon distincte et au-dessus de l'alimentation de la coupe C7+ issue du fond de la colonne de stabilisation C11.

L'effluent issu de la tête de la colonne de purification C6 pouvant être d'un débit très faible par rapport au deux autre flux traités par la section de séparation F, selon un ou plusieurs modes de réalisation de la présente invention, ledit effluent est mélangé avec la coupe C7+ issue du fond de la colonne C11. Toute autre position d'alimentation du flux issu de la tête de la colonne de purification C6 dans la section de séparation F est possible. Par exemple, la tête de la colonne de purification C6 peut être recyclée vers la colonne de benzène C9 ou directement vers l'unité de transalkylation P4. On peut par exemple alimenter l'unité de transalkylation P4 avec un mélange comprenant le produit de fond de la colonne de benzène C9 et un produit de tête de la colonne de purification C6.

Selon un ou plusieurs modes de réalisation, les conditions opératoires de la colonne de toluène C10 sont les suivantes :
température de 130°C à 260°C au rebouilleur, de 50°C à 200°C au condenseur ;
pression : entre 0,05 Mpa et 1 Mpa ; et
entre 30 et 55 plateaux théoriques.

### Colonne de benzène C9 selon le procédé et le dispositif de séparation selon la présente invention

En référence à la figure 2, la coupe C7- extraite en tête de la colonne de toluène C10 (*i.e.,* produit de tête de la colonne de toluène enrichi en benzène et en toluène) est dirigée via la ligne 91 vers la colonne de benzène C9.

Selon un ou plusieurs modes de réalisation de la présente invention, par exemple lorsque la coupe C6-C7 issue de l'unité d'extraction des aromatiques P1 contient très peu de C8+ (par exemple <1% poids de C8+ dans la coupe C6-C7, de façon préférée <0,5% poids et encore plus préférée <0,3% poids), la coupe C6-C7 issue de l'unité d'extraction des aromatiques P1 est dirigée vers la colonne de benzène C9 en aval de la colonne de toluène C10 (en mélange avec ou séparément de la tête de la colonne de toluène C10). Selon un ou plusieurs modes de réalisation, l'alimentation de la colonne de benzène C9 issue de l'unité d'extraction des aromatiques P1 est effectuée séparément et préférablement au-dessus (en aval) de l'alimentation issue de la tête de la colonne de toluène C10.

A partir de la colonne de benzène C9, le produit de tête enrichi en benzène est extrait en tant que produit final via la ligne 90. Selon un ou plusieurs modes de réalisation, le produit enrichi en benzène est extrait par soutirage latéral. Selon un ou plusieurs modes de réalisation, des composés légers et incondensables sont purgés via un flux vapeur en tête de colonne (ligne 92). Le produit de fond de la colonne de benzène C9 enrichi en toluène est dirigé via la ligne 100 vers l'unité de transalkylation P4.

Selon un ou plusieurs modes de réalisation, les conditions opératoires de la colonne de benzène C9 sont les suivantes :
température de 130°C à 180°C au rebouilleur, de 70°C à 130°C au condenseur ;
pression : entre 0,1 Mpa et 0,5 Mpa ; et
entre 35 et 55 plateaux théoriques.

Dans les figures 1 et 2, par soucis de simplification, les sections de reflux, ballons de reflux ou condenseurs ne sont pas représentés ; tout moyen connu de condensation (ex : aéroréfrigérant et/ou réfrigérant à eau) peut être utilisé.

### Exemples

Dans les exemples la section de séparation F est alimentée par les deux charges suivantes :
- Charge 1 : la coupe C6-C7 issue de l'unité d'extraction des aromatiques P1 ; et
- Charge 2 : la coupe C7+ issue du fond de la colonne de stabilisation C11.

Dans les exemples, la tête de la colonne de purification C6 est recyclée directement à l'unité de transalkylation P4.

### Exemple 1

Les compositions des deux charges (Charge 1 et Charge 2) de la section de séparation F sont indiquées dans le tableau 1.

**Tableau 1**

| | | Charge 1 | Charge 2 |
|---|---|---|---|
| BENZENE | kg/hr | 21406 | 12297 |
| Autres C6 | kg/hr | 0 | 27,2 |
| TOLUÈNE | kg/hr | 45521 | 62585 |
| Autres C7 | kg/hr | 0,1 | 15 |
| ETHYL BENZENE | kg/hr | 130 | 339 |
| XYLENES | kg/hr | 636 | 51744 |
| Autres C8 | kg/hr | 0,9 | 0,9 |
| C9+ | kg/hr | 0 | 18682 |

Les coupes en sortie de la section de séparation F sont des produits enrichis en benzène, toluène et C8+, respectivement, selon les compositions du tableau 2.

**Tableau 2**

| | | Produit enrichi en benzène | Produit enrichi en toluène | Produit enrichi en C8+ |
|---|---|---|---|---|
| BENZENE | kg/hr | 33534 | 168 | 0 |
| Autres C6 | kg/hr | 27 | 0 | 0 |
| TOLUÈNE | kg/hr | 1 | 107842 | 263 |
| Autres C7 | kg/hr | 0 | 15 | 0 |
| ETHYL BENZENE | kg/hr | 0 | 9 | 460 |
| XYLENES | kg/hr | 0 | 256 | 52124 |
| Autres C8 | kg/hr | 0 | 2 | 0 |
| C9+ | kg/hr | 0 | 0 | 18682 |

Quatre configurations de fractionnement sont évaluées :
- Configuration 1 (selon le procédé et le dispositif de séparation de référence) : la colonne de benzène est alimentée par le mélange des deux charges. Le fond de la colonne de benzène alimente la colonne de toluène.
- Configuration 2 (comparatif) : la colonne de benzène est alimentée par les deux charges introduites séparément dans la colonne. Le fond de la colonne de benzène alimente la colonne de toluène.
- Configuration 3 (comparatif) : la colonne de toluène est alimentée par le mélange des deux charges. La tête de la colonne de toluène alimente la colonne de benzène.
- Configuration 4 (selon le procédé et le dispositif de séparation selon l'invention) : la colonne de toluène est alimentée par les deux charges introduites séparément dans la colonne. La Charge 1 est alimentée au-dessus de la Charge 2. La tête de la colonne de toluène alimente la colonne de benzène.

Les positions des alimentations sont optimisées dans chacune des configurations de façon à minimiser les consommations énergétiques de rebouillage de chacune des colonnes.

Les géométries et consommations énergétiques de chacune des configurations sont indiquées dans le tableau 3.

On remarque que, de façon surprenante, ce n'est qu'en inversant l'ordre des colonnes et en introduisant séparément les deux charges (configuration 4 selon l'invention) que l'on obtient un gain énergétique significatif (supérieur à 10%) par rapport à l'exemple de référence. La simple inversion des colonnes (configuration 3) ou l'introduction séparée des deux alimentations (configuration 2), réalisées de façon indépendante l'une de l'autre, ne permet pas d'obtenir un gain énergétique important.

Dans cet exemple, il est choisi de diriger la coupe C6-C7 issue de l'unité d'extraction des aromatiques P1 directement vers la colonne de benzène C9 lorsque celle-ci est en aval de la colonne de toluène C10 car sa teneur en C8+ (767 kg/hr) est supérieure à la teneur en C8+ recherchée dans le produit de fond de la colonne de toluène C10 (267kg/hr).

**Tableau 3**

| | Configuration 1 | Configuration 2 | Configuration 3 | Configuration 4 |
|---|---|---|---|---|
| Alimentation de la colonne de benzène C9 | Charge 1 et 2 mélangées | Charge 1 et 2 séparées | Tête de la colonne de toluène | Tête de la colonne de toluène |
| Nombre de plateaux colonne de benzène C9 | 38 | 38 | 38 | 38 |
| Plateau d'alimentation colonne de benzène C9 | 22 | Charge 1 : 20 Charge 2 : 24 | 22 | 22 |
| Alimentation de la colonne de toluène C10 | Fond de la colonne de benzène | Fond de la colonne de benzène | Charge 1 et 2 mélangées | Charge 1 et 2 séparées |
| Nombre de plateaux colonne de toluène C10 | 30 | 30 | 30 | 30 |
| Plateau d'alimentation colonne de toluène C10 | 16 | 16 | 17 | Charge 1 : 5 Charge 2: 18 |
| Consommation énergétique au rebouilleur de la colonne de benzène C9 (MW) | 23,9 | 22,6 | 22,1 | 22,1 |
| Consommation énergétique au rebouilleur de la colonne de toluène C10 (MW) | 29,8 | 29,8 | 33 | 24,2 |
| Consommation énergétique totale des rebouilleurs (MW) | 53,7 | 52,4 | 55,1 | 46,3 |

### Exemple 2

Les compositions des 2 charges à la section de séparation benzène, toluène, C8+ (F) sont indiquées dans le tableau 4.

**Tableau 4**

| | | Charge 1 | Charge 2 |
|---|---|---|---|
| BENZENE | kg/hr | 21406 | 12297 |
| Autres C6 | kg/hr | 0 | 27,2 |
| TOLUÈNE | kg/hr | 45521 | 62585 |
| Autres C7 | kg/hr | 0 | 15 |
| ETHYL BENZENE | kg/hr | 32 | 339 |
| XYLENES | kg/hr | 159 | 51744 |
| Autres C8 | kg/hr | 1 | 0,9 |
| C9+ | kg/hr | 0 | 18682 |

Dans cet exemple la teneur en C8+ (192 kg/hr) dans la Charge 1 permet, dans le schéma selon l'invention où la colonne de benzène C9 est en aval de la colonne de toluène C10, de diriger directement la Charge 1 issue de la coupe C6/C7 issue de l'unité d'extraction des aromatiques P1 vers la colonne de benzène C9.

Trois configurations de fractionnement sont évaluées :
- Configuration 1 (selon le procédé et le dispositif de séparation de référence) : la colonne de benzène est alimentée par le mélange des 2 charges. Le fond de la colonne de benzène alimente la colonne de toluène
- Configuration 5 (selon l'invention) : la colonne de toluène est alimentée par la Charge 2 uniquement. La Charge 1 est mélangée à la tête de la colonne de toluène. Ce mélange alimente la colonne de benzène.
- Configuration 6 (selon l'invention) : la colonne de toluène est alimentée par la Charge 2 uniquement. La Charge 1 et la tête de la colonne de toluène alimentent la colonne de benzène séparément.

Les coupes en sortie de la section de séparation F sont des produits enrichis en benzène, toluène et C8+, respectivement, selon les compositions du tableau 5 selon la configuration 1 et du tableau 6 selon les configurations 5 et 6.

**Tableau 5**

| | | Produit enrichi en benzène | Produit enrichi en toluène | Produit enrichi en C8+ |
|---|---|---|---|---|
| BENZENE | kg/hr | 33534 | 168 | 0 |
| Autres C6 | kg/hr | 27 | 0 | 0 |
| TOLUÈNE | kg/hr | 1 | 107845 | 260 |
| Autres C7 | kg/hr | 0 | 15 | 0 |
| ETHYL BENZENE | kg/hr | 0 | 7 | 364 |
| XYLENES | kg/hr | 0 | 259 | 51644 |
| Autres C8 | kg/hr | 0 | 2 | 0 |
| C9+ | kg/hr | 0 | 0 | 18682 |

**Tableau 6**

| | | Produit enrichi en benzène | Produit enrichi en toluène | Produit enrichi en C8+ |
|---|---|---|---|---|
| BENZENE | kg/hr | 33534 | 168 | 0 |
| Autres C6 | kg/hr | 27 | 0 | 0 |
| TOLUÈNE | kg/hr | 1 | 107844 | 260 |
| Autres C7 | kg/hr | 0 | 15 | 0 |
| ETHYL BENZENE | kg/hr | 0 | 42 | 338 |
| XYLENES | kg/hr | 0 | 224 | 51721 |
| Autres C8 | kg/hr | 0 | 2 | 0 |
| C9+ | kg/hr | 0 | 0 | 18682 |

La teneur en C8+ dans la Charge 1 est suffisamment faible pour que l'on puisse obtenir la même teneur en C8+ dans le produit enrichi en toluène dans les configurations 5 et 6 (268 kg/hr) que dans la configuration 1.

Les positions des alimentations sont optimisées dans chacune des configurations de façon à minimiser les consommations énergétiques de rebouillage de chacune des colonnes.

Les géométries et consommations énergétiques de chacune des configurations sont indiquées dans le tableau 7.

**Tableau 7**

| | Configuration 1 | Configuration 5 | Configuration 6 |
|---|---|---|---|
| Alimentation de la colonne de benzène C9 | Charge 1 et 2 mélangées | Tête de la colonne de toluène et Charge 1 mélangées | Tête de la colonne de toluène et Charge 1 séparées |
| Nombre de plateaux colonne de benzène C9 | 38 | 38 | 38 |
| Plateau d'alimentation colonne de benzène C9 | 22 | 22 | Tête de la colonne de toluène : 28 Charge 1: 22 |
| Alimentation de la colonne de toluène C10 | Fond de la colonne de benzène | Charge 2 | Charge 2 |
| Nombre de plateaux colonne de toluène C10 | 30 | 30 | 30 |
| Plateau d'alimentation colonne de toluène C10 | 16 | 18 | 18 |
| Consommation énergétique au rebouilleur de la colonne de benzène C9 (MW) | 23,9 | 21,7 | 18,8 |
| Consommation énergétique au rebouilleur de la colonne de toluène C10 (MW) | 29,7 | 24,0 | 24,0 |
| Consommation énergétique totale des rebouilleurs (MW) | 53,6 | 45,7 | 42,81 |

Les configurations 5 et 6 selon l'invention sont plus efficaces énergétiquement que configuration 1 selon le procédé et le dispositif de séparation de référence.

## Revendications

1. Procédé de séparation d'une charge comprenant du benzène, du toluène et des composés à 8 atomes de carbone ou plus, dans un dispositif de séparation comprenant au moins une colonne de reformat (C1), une unité d'extraction des aromatiques (P1) et une unité de transalkylation (P4), les effluents desdites unités étant séparés dans les colonnes à distiller suivantes : colonne de benzène (C9), colonne de toluène (C10) et colonne de stabilisation (C11), ledit procédé comprenant les étapes suivantes :
on alimente directement la colonne de toluène (C10) avec une coupe C7+ issue du fond de la colonne de stabilisation (C11) disposée en aval de l'unité de transalkylation (P4) pour soutirer un produit de tête de la colonne de toluène (C10) enrichi en benzène et en toluène et un produit de fond de la colonne de toluène (C10) enrichi en composés à 8 atomes de carbone ou plus,
on alimente la colonne de benzène (C9) avec le produit de tête de la colonne de toluène (C10) pour soutirer un produit de tête de la colonne de benzène (C9) enrichi en benzène et un produit de fond de la colonne de benzène (C9) enrichi en toluène, et
on alimente l'unité de transalkylation (P4) avec le produit de fond de la colonne de benzène (C9) enrichi en toluène,
dans lequel on alimente la colonne de toluène (C10) avec une coupe essentiellement aromatique issue de l'unité d'extraction des aromatiques (P1), l'alimentation de la colonne de toluène (C10) issue de l'unité d'extraction des aromatiques (P1) étant effectuée séparément et au-dessus de l'alimentation de la colonne de toluène (C10) issue du fond de la colonne de stabilisation (C11), ou
dans lequel on alimente la colonne de benzène (C9) avec la coupe essentiellement aromatique issue de l'unité d'extraction des aromatiques (P1) en mélange avec ou séparément du produit de tête de la colonne de toluène (C10).

2. Procédé de séparation selon la revendication 1, dans lequel on alimente en outre la colonne de toluène (C10) avec un produit de tête d'une colonne de purification (C6) en mélange avec ou séparément de la coupe C7+ issue du fond de la colonne de stabilisation (C11).

3. Procédé de séparation selon la revendication 1, dans lequel on alimente en outre l'unité de transalkylation (P4) avec un produit de tête d'une colonne de purification (C6) en mélange avec ou séparément du produit de fond de la colonne de benzène (C9).

4. Procédé de séparation selon l'une quelconque des revendications précédentes, dans lequel le dispositif de séparation comprend en outre une unité de séparation du paraxylène (P2) et une unité d'isomérisation des xylènes (P3), lesdits effluents étant séparés dans les colonnes à distiller additionnelles suivantes : colonne des xylènes (C2), colonne des aromatiques lourds (C3), colonne de raffinat (C4), colonne d'extrait (C5), colonne de purification (C6), déheptaniseur (C7) et stripeur (C8).

5. Procédé de séparation selon la revendication 4 dans lequel seulement la colonne des xylènes (C2) et la colonne des aromatiques lourds (C3) alimentent l'unité de transalkylation (P4) en composés aromatiques en C9+.

6. Procédé de séparation selon l'une quelconque des revendications précédentes, dans lequel : on alimente la colonne de reformat (C1) avec la charge pour produire une coupe C7- en tête de la colonne de reformat (C1), et on alimente l'unité d'extraction des aromatiques (P1) avec la coupe C7- pour extraire des composés paraffiniques de la coupe C7- et produire la coupe essentiellement aromatique.

7. Procédé de séparation selon l'une quelconque des revendications précédentes, les conditions opératoires de la colonne de benzène C9 sont les suivantes :
température de 130°C à 180°C au rebouilleur, de 70°C à 130°C au condenseur ;
pression : entre 0,1 Mpa et 0,5 Mpa ; et
la colonne de benzène C9 comprenant entre 35 et 55 plateaux théoriques.

8. Procédé de séparation selon l'une quelconque des revendications précédentes, les conditions opératoires de la colonne de toluène C10 sont les suivantes :
température de 130°C à 260°C au rebouilleur, de 50°C à 200°C au condenseur ;
pression : entre 0,05 Mpa et 1 Mpa ; et
la colonne de toluène C10 comprenant entre 30 et 55 plateaux théoriques.

9. Dispositif de séparation d'une charge comprenant du benzène, du toluène et des composés à 8 atomes de carbone ou plus, le dispositif de séparation comprenant au moins une colonne de reformat (C1), une unité d'extraction des aromatiques (P1) et une unité de transalkylation (P4), le dispositif de séparation comprenant en outre les colonnes suivantes pour la distillation des effluents desdites unités : colonne de benzène (C9), colonne de toluène (C10) et colonne de stabilisation (C11),
la colonne de toluène (C10) étant adaptée pour être alimentée avec une coupe C7+ issue du fond de la colonne de stabilisation (C11) disposée en aval de l'unité de transalkylation (P4) et pour distribuer un produit de tête de la colonne de toluène (C10) enrichi en benzène et en toluène et un produit de fond de la colonne de toluène (C10) enrichi en composés à 8 atomes de carbone ou plus,
- la colonne de benzène (C9) étant adaptée pour être alimentée avec le produit de tête de la colonne de toluène (C10) et pour distribuer un produit de tête de la colonne de benzène (C9) enrichi en benzène et un produit de fond de la colonne de benzène (C9) enrichi en toluène, et
- l'unité de transalkylation (P4) étant adaptée pour traiter le produit de fond de la colonne de benzène (C9) enrichi en toluène,
dans lequel la colonne de toluène (C10) est adaptée pour être alimentée avec une coupe essentiellement aromatique issue de l'unité d'extraction des aromatiques (P1), l'alimentation de la colonne de toluène (C10) issue de l'unité d'extraction des aromatiques (P1) étant effectuée séparément et au-dessus de l'alimentation de la colonne de toluène (C10) issue du fond de la colonne de stabilisation (C11), ou
dans lequel la colonne de benzène (C9) est adaptée pour être alimentée avec la coupe essentiellement aromatique issue de l'unité d'extraction des aromatiques (P1) en mélange avec ou séparément du produit de tête de la colonne de toluène (C10).

10. Dispositif de séparation selon la revendication 9, dans lequel la colonne de toluène (C10) est adaptée pour être alimentée avec un produit de tête d'une colonne de purification (C6) en mélange avec ou séparément de la coupe C7+ issue du fond de la colonne de stabilisation (C11).

11. Dispositif de séparation selon la revendication 9, dans lequel l'unité de transalkylation (P4) est adaptée pour être alimentée avec un produit de tête d'une colonne de purification (C6) en mélange avec ou séparément du produit de fond de la colonne de benzène (C9).

12. Dispositif de séparation selon l'une quelconque des revendications 9 à 11, comprenant en outre une unité de séparation du paraxylène (P2), une unité d'isomérisation des xylènes (P3) et les colonnes additionnelles suivantes pour la distillation desdits effluents : colonne des xylènes (C2), colonne des aromatiques lourds (C3), colonne de raffinat (C4), colonne d'extrait (C5), colonne de purification (C6), déheptaniseur (C7) et stripeur (C8).

13. Dispositif de séparation selon l'une quelconque des revendications 9 à 12, dans lequel la colonne de reformat (C1) est adaptée pour être alimentée avec la charge pour produire une coupe C7- en tête de la colonne de reformat (C1), et l'unité d'extraction des aromatiques (P1) est adaptée pour être alimentée avec la coupe C7- pour extraire des composés paraffiniques de la coupe C7- et produire la coupe essentiellement aromatique.

14. Dispositif de séparation selon l'une quelconque des revendications 9 à 13, dans lequel la colonne de benzène C9 est adaptée pour être utilisée dans les conditions opératoires suivantes :
température de 130°C à 180°C au rebouilleur, de 70°C à 130°C au condenseur ;
pression : entre 0,1 Mpa et 0,5 Mpa ; et
la colonne de benzène C9 comprenant entre 35 et 55 plateaux théoriques.

15. Dispositif de séparation selon l'une quelconque des revendications 9 à 14, dans lequel la colonne de toluène C10 est adaptée pour être utilisée dans les conditions opératoires suivantes :
température de 130°C à 260°C au rebouilleur, de 50°C à 200°C au condenseur ;
pression : entre 0,05 Mpa et 1 Mpa ; et
la colonne de toluène C10 comprenant entre 30 et 55 plateaux théoriques.

## Patentansprüche

1. Verfahren zur Trennung eines Einsatzmaterials, das Benzol, Toluol und Verbindungen mit 8 oder mehr Kohlenstoffatomen umfasst, in einer Vorrichtung zur Trennung, die mindestens eine Reformatkolonne (C1), eine Einheit zur Extraktion von Aromaten (P1) und eine Transalkylierungseinheit (P4) umfasst, wobei die aus diesen Einheiten abfließenden Ströme in folgenden Destillationskolonnen getrennt werden: Benzolkolonne (C9), Toluolkolonne (C10) und Stabilisierungskolonne (C11), wobei das Verfahren folgende Schritte umfasst:
der Toluolkolonne (C10) wird direkt eine C7+-Fraktion aus dem Sumpf der Stabilisierungskolonne (C11) zugeführt, die in Strömungsrichtung hinter der Transalkylierungseinheit (P4) angeordnet sind, damit ein Kopfprodukt der Toluolkolonne (C10), das mit Benzol und Toluol angereichert ist, und ein Sumpfprodukt der Toluolkolonne (C10) abgezogen wird, das mit Verbindungen mit 8 oder mehr Kohlenstoffatomen angereichert ist,
der Benzolkolonne (C9) wird das Kopfprodukt der Toluolkolonne (C10) zugeführt, damit ein Kopfprodukt der Benzolkolonne (C9), das mit Benzol angereichert ist, und ein Sumpfprodukt der Benzolkolonne (C9) abgezogen wird, das mit Toluol angereichert ist, und
der Transalkylierungseinheit (P4) wird das Sumpfprodukt der Benzolkolonne (C9) zugeführt, das mit Toluol angereichert ist,
wobei der Toluolkolonne (C10) eine im Wesentlichen aromatische Fraktion aus der Einheit zur Extraktion von Aromaten (P1) zugeführt, wobei die Zufuhr zur Toluolkolonne (C10) aus der Einheit zur Extraktion von Aromaten (P1) getrennt von der und über der Zufuhr zur Toluolkolonne (C10) vom Sumpf der Stabilisierungskolonne (C11) her erfolgt, oder
wobei der Benzolkolonne (C9) die im Wesentlichen aromatische Fraktion aus der Einheit zur Extraktion von Aromaten (P1) gemischt mit oder getrennt von dem Kopfprodukt der Toluolkolonne (C10) zugeführt wird.

2. Verfahren zur Trennung nach Anspruch 1, wobei ferner der Toluolkolonne (C10) ein Kopfprodukt einer Reinigungskolonne (C6) gemischt mit der oder getrennt von der aus dem Sumpf der Stabilisierungskolonne (C11) stammenden C7+-Fraktion zugeführt wird.

3. Verfahren zur Trennung nach Anspruch 1, wobei ferner der Transalkylierungseinheit (P4) ein Kopfprodukt einer Reinigungskolonne (C6) gemischt mit dem oder getrennt von dem Sumpfprodukt der Benzolkolonne (C9) zugeführt wird.

4. Verfahren zur Trennung nach einem der vorhergehenden Ansprüche, wobei die Vorrichtung zur Trennung ferner eine Einheit zur Trennung von Paraxylen (P2) und eine Einheit zur Isomerisierung von Xylenen (P3) umfasst, wobei die abfließenden Ströme in folgenden zusätzlichen Destillationskolonnen getrennt werden: Xylenkolonne (C2), Kolonne für schwere Aromaten (C3), Raffinatkolonne (C4), Extraktkolonne (C5), Reinigungskolonne (C6), Deheptanisator (C7) und Stripper (C8).

5. Verfahren zur Trennung nach Anspruch 4, wobei der Transalkylierungseinheit (P4) ausschließlich aus der Xylenkolonne (C2) und der Kolonne für schwere Aromaten (C3) aromatische C9+-Verbindungen zugeführt werden.

6. Verfahren zur Trennung nach einem der vorhergehenden Ansprüche, wobei: der Reformatkolonne (C1) das Einsatzmaterial zugeführt wird, damit am Kopf der Reformatkolonne (C1) eine Fraktion C7- erzeugt wird, und der Einheit zur Extraktion von Aromaten (P1) die Fraktion C7- zugeführt wird, damit aus der Fraktion C7-paraffinhaltige Verbindungen herausgelöst werden und die im Wesentlichen aromatische Fraktion erzeugt wird.

7. Verfahren zur Trennung nach einem der vorhergehenden Ansprüche, wobei die Betriebsbedingungen der Benzolkolonne (C9) folgendermaßen lauten:
Temperatur am Rückverdampfer von 130°C bis 180°C, am Kondensator von 70°C bis 130°C;
Druck: zwischen 0,1 MPa und 0,5 MPa; und
wobei die Benzolkolonne (C9) zwischen 35 und 55 theoretische Böden umfasst.

8. Verfahren zur Trennung nach einem der vorhergehenden Ansprüche, wobei die Betriebsbedingungen der Toluolkolonne (C10) folgendermaßen lauten:
Temperatur am Rückverdampfer von 130°C bis 260°C, am Kondensator von 50°C bis 200°C;
Druck: zwischen 0,05 MPa und 1 MPa; und
wobei die Toluolkolonne (C10) zwischen 30 und 55 theoretische Böden umfasst.

9. Vorrichtung zur Trennung eines Einsatzmaterials, das Benzol, Toluol und Verbindungen mit 8 oder mehr Kohlenstoffatomen umfasst, wobei die Vorrichtung zur Trennung mindestens eine Reformatkolonne (C1), eine Einheit zur Extraktion von Aromaten (P1) und eine Transalkylierungseinheit (P4) umfasst, wobei die Vorrichtung zur Trennung ferner folgende Kolonnen für die Destillation der aus diesen Einheiten abfließenden Ströme umfasst: Benzolkolonne (C9), Toluolkolonne (C10) und Stabilisierungskolonne (C11),
wobei die Toluolkolonne (C10) so ausgelegt ist, dass ihr eine C7+-Fraktion aus dem Sumpf der Stabilisierungskolonne (C11) zugeführt, die in Strömungsrichtung hinter der Transalkylierungseinheit (P4) angeordnet ist, und dass sie ein Kopfprodukt der Toluolkolonne (C10), das mit Benzol und Toluol angereichert ist, und ein Sumpfprodukt der Toluolkolonne (C10) abgibt, das mit Verbindungen mit 8 oder mehr Kohlenstoffatomen angereichert ist,
- wobei die Benzolkolonne (C9) so ausgelegt ist, dass ihr das Kopfprodukt der Toluolkolonne (C10) zugeführt wird und dass sie ein Kopfprodukt der Benzolkolonne (C9), das mit Benzol angereichert ist, und ein Sumpfprodukt der Benzolkolonne (C9) abgibt, das mit Toluol angereichert ist, und
- wobei die Transalkylierungseinheit (P4) so ausgelegt ist, dass sie das Sumpfprodukt der Benzolkolonne (C9) behandelt, das mit Toluol angereichert ist,
wobei die Toluolkolonne (C10) so ausgelegt ist, dass ihr eine im Wesentlichen aromatische Fraktion aus der Einheit zur Extraktion von Aromaten (P1) zugeführt wird, wobei die Zufuhr zur Toluolkolonne (C10) aus der Einheit zur Extraktion von Aromaten (P1) getrennt von der und über der Zufuhr zur Toluolkolonne (C10) vom Sumpf der Stabilisierungskolonne (C11) her erfolgt, oder
wobei die Benzolkolonne (C9) so ausgelegt ist, dass ihr die im Wesentlichen aromatische Fraktion aus der Einheit zur Extraktion von Aromaten (P1) gemischt mit oder getrennt von dem Kopfprodukt der Toluolkolonne (C10) zugeführt wird.

10. Vorrichtung zur Trennung nach Anspruch 9, wobei die Toluolkolonne (C10) so ausgelegt ist, dass ihr ein Kopfprodukt einer Reinigungskolonne (C6) gemischt mit der oder getrennt von der aus dem Sumpf der Stabilisierungskolonne (C11) stammenden C7+-Fraktion zugeführt wird.

11. Vorrichtung zur Trennung nach Anspruch 9, wobei die Transalkylierungseinheit (P4) so ausgelegt ist, dass ihr ein Kopfprodukt einer Reinigungskolonne (C6) gemischt mit dem oder getrennt von dem Sumpfprodukt der Benzolkolonne (C9) zugeführt wird.

12. Vorrichtung zur Trennung nach einem der Ansprüche 9 bis 11, die ferner eine Einheit zur Trennung von Paraxylen (P2), eine Einheit zur Isomerisierung von Xylenen (P3) und folgende zusätzliche Kolonnen für die Destillation der abfließenden Ströme umfasst: Xylenkolonne (C2), Kolonne für schwere Aromaten (C3), Raffinatkolonne (C4), Extraktkolonne (C5), Reinigungskolonne (C6), Deheptanisator (C7) und Stripper (C8).

13. Vorrichtung zur Trennung nach einem der Ansprüche 9 bis 12, wobei die Reformatkolonne (C1) so ausgelegt ist, dass ihr das Einsatzmaterial zugeführt wird, damit am Kopf der Reformatkolonne (C1) eine Fraktion C7- erzeugt wird, und die Einheit zur Extraktion von Aromaten (P1) so ausgelegt ist, dass ihr die Fraktion C7- zugeführt wird, damit aus der Fraktion C7- paraffinhaltige Verbindungen herausgelöst werden und die im Wesentlichen aromatische Fraktion erzeugt wird.

14. Vorrichtung zur Trennung nach einem der Ansprüche 9 bis 13, wobei die Benzolkolonne (C9) so ausgelegt ist, dass sie unter folgenden Betriebsbedingungen eingesetzt wird:
Temperatur am Rückverdampfer von 130°C bis 180°C, am Kondensator von 70°C bis 130°C;
Druck: zwischen 0,1 MPa und 0,5 MPa; und
wobei die Benzolkolonne (C9) zwischen 35 und 55 theoretische Böden umfasst.

15. Vorrichtung zur Trennung nach einem der Ansprüche 9 bis 14, wobei die Toluolkolonne (C10) so ausgelegt ist, dass sie unter folgenden Betriebsbedingungen eingesetzt wird:
Temperatur am Rückverdampfer von 130°C bis 260°C, am Kondensator von 50°C bis 200°C;
Druck: zwischen 0,05 MPa und 1 MPa; und
wobei die Toluolkolonne (C10) zwischen 30 und 55 theoretische Böden umfasst.

## Claims

1. Process for the separation of a feedstock comprising benzene, toluene and compounds having 8 or more carbon atoms, in a separation device comprising at least one reformate column (C1), one aromatics extraction unit (P1) and one transalkylation unit (P4), the effluents from the said units being separated in the following distillation columns: benzene column (C9), toluene column (C10) and stabilization column (C11), the said process comprising the following stages:
the toluene column (C10) is fed directly with a C7+ cut resulting from the bottom of the stabilization column (C11) positioned downstream of the transalkylation unit (P4) in order to withdraw a top product from the toluene column (C10) enriched in benzene and in toluene and a bottom product from the toluene column (C10) enriched in compounds having 8 or more carbon atoms,
the benzene column (C9) is fed with the top product from the toluene column (C10) in order to withdraw a benzene-enriched top product from the benzene column (C9) and a toluene-enriched bottom product from the benzene column (C9), and
the transalkylation unit (P4) is fed with the toluene-enriched bottom product from the benzene column (C9),
in which the toluene column (C10) is fed with an essentially aromatic cut resulting from the aromatics extraction unit (P1), the feeding of the toluene column (C10) resulting from the aromatics extraction unit (P1) being carried out separately from and above the feeding of the toluene column (C10) resulting from the bottom of the stabilization column (C11), or
in which the benzene column (C9) is fed with the essentially aromatic cut resulting from the aromatics extraction unit (P1) as a mixture with or separately from the top product from the toluene column (C10).

2. Separation process according to Claim 1, in which the toluene column (C10) is additionally fed with a top product from a purification column (C6) as a mixture with or separately from the C7+ cut resulting from the bottom of the stabilization column (C11).

3. Separation process according to Claim 1, in which the transalkylation unit (P4) is additionally fed with a top product from a purification column (C6) as a mixture with or separately from the bottom product from the benzene column (C9).

4. Separation process according to any one of the preceding claims, in which the separation device additionally comprises a para-xylene separation unit (P2) and a xylenes isomerization unit (P3), the said effluents being separated in the following additional distillation columns: xylenes column (C2), heavy aromatics column (C3), raffinate column (C4), extract column (C5), purification column (C6), deheptanizer (C7) and stripper (C8).

5. Separation process according to Claim 4, in which only the xylenes column (C2) and the heavy aromatics column (C3) feed the transalkylation unit (P4) with C9+ aromatic compounds.

6. Separation process according to any one of the preceding claims, in which: the reformate column (C1) is fed with the feedstock in order to produce a C7- cut at the top of the reformate column (C1), and the aromatics extraction unit (P1) is fed with the C7-cut in order to extract paraffinic compounds from the C7- cut and to produce the essentially aromatic cut.

7. Separation process according to any one of the preceding claims, in which the operating conditions of the benzene column C9 are as follows:
temperature of 130°C to 180°C at the reboiler, from 70°C to 130°C at the condenser;
pressure: between 0.1 MPa and 0.5 MPa; and
the benzene column C9 comprising between 35 and 55 theoretical plates.

8. Separation process according to any one of the preceding claims, in which the operating conditions of the toluene column C10 are as follows:
temperature of 130°C to 260°C at the reboiler, from 50°C to 200°C at the condenser;
pressure: between 0.05 MPa and 1 MPa; and
the toluene column C10 comprising between 30 and 55 theoretical plates.

9. Device for the separation of a feedstock comprising benzene, toluene and compounds having 8 or more carbon atoms, the separation device comprising at least one reformate column (C1), one aromatics extraction unit (P1) and one transalkylation unit (P4), the separation device additionally comprising the following columns for the distillation of the effluents from the said units: benzene column (C9), toluene column (C10) and stabilization column (C11),
the toluene column (C10) being appropriate for being fed with a C7+ cut resulting from the bottom of the stabilization column (C11) positioned downstream of the transalkylation unit (P4) and for delivering a top product from the toluene column (C10) enriched in benzene and in toluene and a bottom product from the toluene column (C10) enriched in compounds having 8 or more carbon atoms,
- the benzene column (C9) being appropriate for being fed with the top product from the toluene column (C10) and for delivering a benzene-enriched top product from the benzene column (C9) and a toluene-enriched bottom product from the benzene column (C9), and
- the transalkylation unit (P4) being appropriate for treating the toluene-enriched bottom product from the benzene column (C9),
in which the toluene column (C10) is appropriate for being fed with an essentially aromatic cut resulting from the aromatics extraction unit (P1), the feeding of the toluene column (C10) resulting from the aromatics extraction unit (P1) being carried out separately from and above the feeding of the toluene column (C10) resulting from the bottom of the stabilization column (C11), or
in which the benzene column (C9) is appropriate for being fed with the essentially aromatic cut resulting from the aromatics extraction unit (P1) as a mixture with or separately from the top product from the toluene column (C10).

10. Separation device according to Claim 9, in which the toluene column (C10) is appropriate for being fed with a top product from a purification column (C6) as a mixture with or separately from the C7+ cut resulting from the bottom of the stabilization column (C11).

11. Separation device according to Claim 9, in which the transalkylation unit (P4) is appropriate for being fed with a top product from a purification column (C6) as a mixture with or separately from the bottom product from the benzene column (C9).

12. Separation device according to any one of Claims 9 to 11, additionally comprising a para-xylene separation unit (P2), a xylenes isomerization unit (P3) and the following additional columns for the distillation of the said effluents: xylenes column (C2), heavy aromatics column (C3), raffinate column (C4), extract column (C5), purification column (C6), deheptanizer (C7) and stripper (C8).

13. Separation device according to any one of Claims 9 to 12, in which the reformate column (C1) is appropriate for being fed with the feedstock in order to produce a C7- cut at the top of the reformate column (C1), and the aromatics extraction unit (P1) is appropriate for being fed with the C7- cut in order to extract paraffinic compounds from the C7- cut and to produce the essentially aromatic cut.

14. Separation device according to any one of Claims 9 to 13, in which the benzene column C9 is appropriate for being used under the following operating conditions:
temperature of 130°C to 180°C at the reboiler, from 70°C to 130°C at the condenser;
pressure: between 0.1 MPa and 0.5 MPa; and
the benzene column C9 comprising between 35 and 55 theoretical plates.

15. Separation device according to any one of Claims 9 to 14, in which the toluene column C10 is appropriate for being used under the following operating conditions:
temperature of 130°C to 260°C at the reboiler, from 50°C to 200°C at the condenser;
pressure: between 0.05 MPa and 1 MPa; and
the toluene column C10 comprising between 30 and 55 theoretical plates.
